(19) Europäisches Patentamt
European Patent Office
Office européen des brevets

(11) **EP 4 159 875 A1**

(12) **EUROPEAN PATENT APPLICATION**
published in accordance with Art. 153(4) EPC

(43) Date of publication:
**05.04.2023  Bulletin 2023/14**

(21) Application number: **20937608.6**

(22) Date of filing: **10.11.2020**

(51) International Patent Classification (IPC):
**C12Q 1/70** (2006.01)    **C12Q 1/686** (2018.01)
**C12N 15/11** (2006.01)    **C12R 1/93** (2006.01)

(86) International application number:
**PCT/CN2020/127752**

(87) International publication number:
**WO 2021/238087 (02.12.2021 Gazette 2021/48)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority:  **28.05.2020  CN 202010465211**

(71) Applicant: **Daan Gene Co., Ltd.**
**Guangdong 510665 (CN)**

(72) Inventors:
• **JIANG, Xiwen**
**Guangzhou, Guangdong 510665 (CN)**

• **HUANG, Taosheng**
**Guangzhou, Guangdong 510665 (CN)**
• **LU, Zhuojian**
**Guangzhou, Guangdong 510665 (CN)**
• **CHEN, Qixian**
**Guangzhou, Guangdong 510665 (CN)**
• **LIN, Weiping**
**Guangzhou, Guangdong 510665 (CN)**

(74) Representative: **Diehl & Partner**
**Patent- und Rechtsanwaltskanzlei mbB**
**Erika-Mann-Straße 9**
**80636 München (DE)**

Remarks:
The complete document including Reference Table(s) and the Sequence Listing(s) can be downloaded from the EPO website

(54) **NOVEL CORONAVIRUS RAPID DETECTION KIT BASED ON THERMAL CONVECTION PCR**

(57)    The present invention provides a novel coronavirus rapid detection kit based on thermal convection PCR and a method for multiple detection of novel coronavirus nucleic acid. The kit comprises a combination of primers and probes for detecting N genes and ORF1ab genes of a novel coronavirus genome.

EP 4 159 875 A1

Fig. 1

**Description**

**Technical field**

The present invention belongs to the field of biotechnology and molecular diagnosis. Specifically, the present invention relates to a novel coronavirus rapid detection kit based on thermal convection PCR.

**Background**

The novel coronavirus (2019 novel corona virus, 2019-nCoV, SARS-CoV-2) belongs to the β-coronavirus genus, with an envelope, and the particles are round or oval, often pleomorphic, with a diameter of 60-140nm. The S protein is one of the main proteins of the virus, and its encoded gene is used for virus typing. The molecular mechanism of the interaction between the S-protein and human ACE2 is used to infect human respiratory epithelial cells. The common signs of people infected with this coronavirus are fever, cough, shortness of breath, difficulty breathing and the like. Severe infections can lead to pneumonia, severe malformed respiratory syndrome, kidney failure, and even death. This new type of coronavirus is highly infectious to humans, and the lung infection it causes is named "Novel coronavirus pneumonia" (COVID-19).

All organisms except prions contain nucleic acids, which include deoxyribonucleic acid (DNA) and ribonucleic acid (RNA). The novel coronavirus is a virus that only contains RNA. The specific RNA sequence in the virus is the marker which distinguishes the virus from other pathogens. After the emergence of the novel coronavirus, scientists completed the analysis of the whole genome sequence of the novel coronavirus in a very short period of time, and found the specific nucleic acid sequence in the novel coronavirus by comparing it with the genome sequences of other species. During clinical laboratory testing, if a specific nucleic acid sequence of the novel coronavirus is detected in a patient sample, it should be suggested that the patient may be infected by the novel coronavirus. The most common method for detecting the specific sequence of the novel coronavirus is fluorescent quantitative PCR (Polymerase Chain Reaction). The PCR reaction template is DNA only, so the novel coronavirus RNA should be reverse transcribed into DNA before performing the PCR reaction. In the PCR reaction system, a pair of specific primers and a Taqman probe are included. The probe is a specific oligonucleotide sequence, and the two ends are respectively labeled with a reporter fluorescent group and a quencher fluorescent group. When the probe is intact, the fluorescent signal emitted by the reporter group is absorbed by the quencher group. If there is a target sequence in the reaction system, during the PCR reaction, the probe binds to the template, then the DNA polymerase degrades the probe along the template using its exonuclease activity, and then the reporter group is separated from the quencher group and fluoresces. For each DNA strand amplified, one fluorescent molecule is produced. The fluorescent quantitative PCR instrument can detect the number of cycles (Ct value) at which the fluorescence reaches the preset threshold which is related to the concentration of viral nucleic acid. The higher the concentration of viral nucleic acid, the smaller the Ct value. The products of different manufacturers will determine the positive judgment value of the novel coronavirus according to the performance of their own products.

The fluorescent quantitative PCR equipment currently used has problems such as high instrument cost and large laboratory operation space. It needs to be submitted for inspection in a centralized manner, the test is carried out in a dedicated PCR laboratory, and rapid on-site inspection cannot be carried out. The experiment time is long, even excluding the nucleic acid processing process, the amplification detection time is generally about 2 hours. Due to above reasons, the current nucleic acid detection kits cannot carry out large-scale community screening, which limits the scope of application of existing nucleic acid detection products and cannot push diagnosis forward and down.

Therefore, it is necessary to develop a rapid detection technology for the novel coronavirus.

**Summary of the invention**

The present invention develops a novel coronavirus rapid diagnosis system for the novel coronavirus genome N gene and ORF1ab gene. In order to be able to detect patients with novel coronavirus infection with high efficiency, high specificity and low cost, and prevent possible variability.

In the first aspect of the present invention, it provides a primer pair set for detection of novel coronavirus nucleic acid comprising:

a first primer pair, wherein the first primer pair comprises:

a forward primer as shown in SEQ ID NO.1, and a reverse primer as shown in SEQ ID NO.2.

In another preferred embodiment, the primer pair set further includes:

a second primer pair, wherein the second primer pair comprises:

a forward primer as shown in SEQ ID NO.4, and a reverse primer as shown in SEQ ID NO.5.

In another preferred embodiment, the primer pair set further includes:

an internal standard primer pair, wherein the internal standard primer pair comprises:

a forward primer as shown in SEQ ID NO.7; and a reverse primer as shown in SEQ ID NO.8.

In the second aspect of the present invention, it provides a probe set for multiplex detection of novel coronavirus nucleic acid, which includes a first probe whose nucleotide sequence is shown in SEQ ID NO. 3.

In another preferred example, the probe set further includes a second probe whose nucleotide sequence is shown in SEQ ID NO.6.

In another preferred embodiment, the probe set further includes an internal control probe whose nucleotide sequence is shown in SEQ ID NO.9.

In another preferred embodiment, the 5' end of each probe is labeled with a fluorescent reporter group; and/or, the 3' end of each probe is labeled with a fluorescence quenching group.

In another preferred embodiment, the fluorescent reporter groups labeled on the probes are different from each other.

In the third aspect of the present invention, it provides a kit for multiplex detection of novel coronavirus nucleic acid, which comprises the primer pair set according to the first aspect of the present invention.

In another preferred embodiment, the kit further comprises the probe set according to the second aspect of the present invention.

In another preferred embodiment, the kit comprises a first container, and the first container contains a primer and probe mix (primer-probe mixture), and the primer and probe mix contains polynucleotides having sequences shown in SEQ ID NOs. 1 to 6; preferably further contains dNTPs.

In another preferred embodiment, and the primer and probe mix further contains polynucleotides having sequences shown in SEQ ID NOs.7 to 9.

In another preferred example, the kit further comprises a second container, and the second container contains a PCR enzyme system including a Hot-start Taq enzyme and Reverse transcriptase C-MMLV.

In another preferred embodiment, the kit further includes a third container, and the third container contains a negative control.

In another preferred embodiment, the kit further includes a fourth container, and the fourth container contains a positive control.

In the fourth aspect of the present invention, it provides a method for multiplex detection of novel coronavirus nucleic acid comprising the following steps:

(1) providing a nucleic acid sample of a subject to be tested;
(2) preparing a PCR reaction system for the PCR detection:
wherein the PCR reaction system includes: the nucleic acid sample provided in step (1), the primer pair set according to the first aspect of the present invention, and the probe set according to the second aspect of the present invention.

In another preferred embodiment, the nucleic acid sample may be from a throat swab sample, a alveolar lavage fluid sample, a blood sample, a sputum sample, a stool sample, or an environmental sample.

In another preferred embodiment, the method is a detection method for non-diagnostic purposes.

In another preferred embodiment, the PCR reaction system further includes a positive control, and/or a negative control.

In another preferred embodiment, the PCR reaction system further includes a PCR enzyme system.

In another preferred embodiment, the PCR is fluorescent quantitative PCR or thermal convection PCR.

In a fifth aspect of the present invention, it provides a use of the primer pair set according to the first aspect of the present invention, and/or the probe set according to the second aspect of the present invention for preparing a PCR detection kit, the PCR detection kit is used to detect novel coronavirus nucleic acid.

In another preferred embodiment, the PCR is fluorescent quantitative PCR or thermal convection PCR.

It should be understood that, within the scope of the present invention, the technical features specifically described above and below (such as the examples) can be combined with each other, thereby constituting a new or preferred technical solution which needs not be described one by one.

**Description of drawings**

Fig. 1: Sensitivity test results;
Fig. 2: Specific test results;
Fig. 3: Repeatable test results;
Fig. 4: Typical clinical sample test results;
Fig. 5: The detection results of the nCoV-N-F2 and nCoV-N-R2;

Fig. 6 and Fig. 7 were the detection results of the nCoV-N-F3 and nCoV-N-R3(Fig. 6 N gene single system; Fig. 7 multiple system);

Fig. 8 and Fig.9 showed the detection results of the nCoV-N-F4 and nCoV-N-R4 in the ordinary fluorescent system (Fig.8) and the thermal convection fast system (Fig.9), respectively.

**Detailed description of invention**

Through extensive and intensive research, the inventors obtained a kit and method for rapid detection of novel coronavirus nucleic acid. After several rounds of screening and verification, the present invention obtains a primer-probe combination with high sensitivity, strong specificity and good repeatability from a large number of primer-probe combinations, which is suitable for thermal convection PCR and ordinary fluorescent quantitative PCR, and capable of multiple detection. Using the kit and detection method of the present invention, based on thermal convection PCR, the ultra-fast PCR amplification detection of 50 cycles can be completed in 18 minutes, and the detection sensitivity is equivalent to that of ordinary fluorescent quantitative PCR.

Before describing the present invention, it should be understood that the present invention is not limited to the specific methods and experimental conditions as described, due to such methods and conditions may vary. It should also be understood that the terminology used herein is for the purpose of describing specific embodiments only and is not intended to be limiting, and the scope of the present invention will be limited only by the appended claims.

Unless otherwise defined, all technical and scientific terms used herein have the same meaning as commonly understood by those of ordinary skill in the art to which this invention belongs. As used herein, when used in reference to specifically recited value, the term "about" means that the value may vary from the recited value by no more than 1%. For example, as used herein, the expression "about 100" includes all values between 99 and 101 and (e.g., 99.1, 99.2, 99.3, 99.4, etc.).

Although any methods and materials similar or equivalent to those described in the present invention can be used in the practice or testing of the present invention, the preferred methods and materials are exemplified herein.

Thermal Convection PCR

Thermal convection PCR is in a closed space, keeping the temperature of its upper and lower surfaces constant, resulting in a regular scrolling pattern phenomenon in which hot fluid rises and cold fluid descends. The heat convection caused by the density change of the fluid in the space caused by the temperature difference between the upper and lower sides is applied to DNA amplification. The lower part of the capillary tube is subjected to constant heating causing the denaturation reaction of the sample at the bottom. At the same time, its density becomes smaller, and the buoyancy gradually increases. When the buoyancy is greater than the viscous force and gravity the sample at the bottom floats upward and rises to a lower temperature region near the top of the tube. The sample undergoes annealing and extension reactions, while being affected by cooling. The density becomes greater again and begins to sink slowly. And the heating and cooling process is repeated. In this way, PCR reactions are achieved by thermal convection.

However, there are also many difficulties in achieving stable amplification using thermal convection PCR. For example, it is difficult to ensure that the thermal convection fluid path is regular, which leads to the inability to ensure that the reaction reagents can fully complete the three steps of deformation, annealing and extension which in turn leads to problems such as low amplification efficiency, poor amplification specificity, and large differences between tubes. Therefore, the design of specific reagents especially primers and probes is more demanding in thermal convection PCR

The thermal convection PCR equipment currently available in the market includes the POCKIT™ nucleic acid analyzer developed by Taiwan GeneReach Biotechnology Corporation and the thermal convection PCR detector developed by Achem Biosystems (Korea).

Multiplex PCR

Multiplex PCR, also known as multiple primers PCR or complex PCR, is a PCR reaction in which two or more pairs of primers are added to the same PCR reaction system to simultaneously amplify multiple nucleic acid fragments. The reaction principle, reaction reagents and operation process are the same as general PCR.

There are many factors that affect multiplex PCR reactions, such as:

(1) The imbalance of the reaction system. The imbalance of the reaction system leads to the rapid amplification of certain dominant primers and their templates in the previous rounds of reactions, and a large number of amplification products are obtained, and these amplification products are also good inhibitors of DNA polymerase. Therefore, as a large number of amplification products appear, the polymerization ability of the polymerase is more and more strongly inhibited. Therefore, the primers and their templates that are at a disadvantage in the early stage are more difficult to react, resulting in a very small amount of amplification product that cannot be detected.

(2) Primer specificity. If the primer has stronger binding ability with other non-target gene fragments in the system, the ability of the target gene to bind to the primer will be contested, resulting in a decrease in amplification efficiency.

(3) The optimal annealing temperature is inconsistent. Multiple pairs of primers are put into a system for amplification. Since the annealing temperature for PCR reaction is the same, the optimal annealing temperature of each pair of primers is required to be close.

(4) Primer dimers, including the dimers between primers and the hairpin structure formed by the primers themselves, and there is also a third-party DNA-mediated polymer. Like non-specific primers, these dimers will interfere with the competition between the primer and the target binding site, affecting the amplification efficiency.

Although several factors affecting the efficiency of amplification are mentioned as above, more factors are unclear. So far, there is no effective method that can clearly predict the amplification efficiency.

The Kit

The present invention provides a kit for specifically detecting the novel coronavirus nucleic acid in a sample, including a combination of primers and probes for detecting N gene, wherein the primer sequences for detecting the N gene are:

SEQ ID NO. 1: GGGGAACTTCTCCTGCTAGAAT and SEQ ID NO. 2: CAGACATTTTGCTCTCAAGCTG, and the corresponding detection probe sequence is SEQ ID NO.3: TTGCTGCTGCTTGACAGATT.

In one embodiment, the kit further includes a combination of primers and probes for detecting the ORF lab gene, wherein the primer sequence for detecting the ORF1ab gene are:

SEQ ID NO.4:CCCTGTGGGTTTTACACTTAA and SEQ ID NO.5: ACGATTGTGCATCAGCTGA, and the sequence of the corresponding detection probe is SEQ ID NO. 6: CCGTCTGCGGTATGTGGAAAGGTTATGG.

In one embodiment, the kit further includes internal standard quality control amplification primers and detection probes; and the sequences of the internal standard quality control amplification primers are:

SEQ ID NO.7: CTCCGTACACTCTCCTACCCTC and SEQ ID NO.8: ACACCTCTGGATGCCACT, and the sequence of the corresponding detection probe is SEQ ID NO. 9: CCATTGCCAGTCCGCCGTC.

In a preferred embodiment of the present invention, the 5' end of each probe is labeled with a fluorescent reporter group; and/or, the 3' end of each probe is labeled with a fluorescence quenching group.

In another preferred embodiment, the fluorescent reporter groups labeled on the probes are different from each other.

Preferably, the fluorescent group is selected from the group consisting of FAM, VIC, HEX, NED, ROX, TET, JOE, TAMRA, CY3, and CY5.

Preferably, the quenching group is selected from the group consisting of MGB, BHQ-1, BHQ-2, and BHQ-3.

In one embodiment, the kit includes a positive quality control (Including pseudoviruses containing target fragments and pseudoviruses containing internal standard fragments) and a negative quality control (Pseudovirus with internal standard fragment).

In one embodiment, the kit includes PCR reaction solution A and PCR reaction solution B, wherein PCR reaction solution A includes specific primers and probes (SEQ ID NO.: 1-9), Tris-HCl Buffer, $Mg^{2+}$ and dNTPs; PCR reaction solution B includes Hot start Taq enzyme, C-MMLV enzyme.

For the gene sequence of the novel coronavirus in the present invention, please refers to GISAID:BetaCov/Wuhan/WH01/2019| EPI_ISL_406798. For the oligonucleotide sequence information of its E gene, please refer to the Reference Roujian Lu, Xiang Zhao, Juan Li, et. al, Genomic characterisation and epidemiology of 2019 novel coronavirus: implications for virus origins and receptor binding. Lancet. 2020 Jan 30.

In present invention, the specific primers and probes are screened by a large number of tests, and then combined, optimized and verified and finally dual primer-probe combinations that will not interfere with each other, have high amplification efficiency, and have good specificity are finally screened.

Detection Methods

1. Sample Processing and Nucleic Acid Extraction

It is recommended to take 200μl liquid sample for nucleic acid extraction. Nucleic acid extraction or purification kits (Magnetic Bead Method) produced by Sun Yat-Sen University Daan Gene Co., Ltd. can be used(Yuesuixiebei No. 20170583 and Yuesuixiebei No. 20150302), and other commercial products can also be used, and the specific operation steps should be carried out according to the instructions of the kit.

Both the negative and positive quality controls in this kit are involved in the extraction.

2. Preparation of PCR reagent

Take out NC(ORF1ab/N) PCR reaction solution A and NC(ORF1ab/N) PCR reaction solution B from the kit, thaw at room temperature, shake and mix well, and centrifuge at 8000rpm for a few seconds before use.

Take N+1 (N=number of samples to be tested + NC(ORF1ab/N) negative quality control + NC(ORF1ab/N) positive quality control) PCR reaction tubes.

The single-person amplification system is prepared in the following table:

| NC(ORF1ab/N) PCR reaction solution A | NC(ORF1ab/N) PCR reaction solution B | Amplification system |
|---|---|---|
| 12 μl | 3 μl | 15 μl |

Take N+1 dedicated PCR reaction tubes, and use a narrow-mouth pipette tip to aliquot the PCR reagents (15 μl per tube). After the aliquoting is completed, use a centrifuge at 8000 rpm for 1 min to allow the PCR reaction solution to flow into the bottom of the PCR reaction tube.

3.Add Sample

The PCR reaction tubes were added with the extracted DNA samples, negative quality control products and positive quality control products of 5μl respectively. Cover the tube cap tightly and centrifuge at 8000 rpm for 3 minutes. After centrifugation, observe whether there are visible bubbles remaining in the reaction tube. Repeat centrifugation if necessary. If there are no visible bubbles, each reaction tube was placed into a portable rapid PCR instrument (Achem Biosystems (Korea)).

4. PCR Amplification

The program was set as follows: first perform reverse transcription at 50°C for 4 minutes, and then perform 50 cycles of thermal convection PCR reaction at 95°C. Select the FAM channel, VIC channel, and CY5 channel for detection.

5. Result Analysis and Judgment

When the instrument was normal, and the NC (ORF1ab/N) positive quality control and NC (ORF1ab/N) negative quality control were both normal, the result analysis was performed.

FAM channel and VIC channel:

5.1 Positive: If the test results of the FAM channel and VIC channel of the sample to be tested were Ct $\leq$ 40 and the curve was S-shaped or there was an obvious exponential growth period, or the FAM channel and VIC had one Ct <_ 40 and the other Ct $\leq$ 45, it was judged as 2019 novel coronavirus ( 2019-nCoV) positive;

5.2 Suspicious: The test results of the FAM channel and VIC channel of the sample to be tested are both 40$\leq$Ct$\leq$45. At this time, the sample should be tested repeatedly. If the Ct value of the rereading result still exists in the same range, the curve was S-shaped or has an obvious exponential expansion period, it was judged that the 2019 novel coronavirus (2019-nCoV) was positive, otherwise the 2019 novel coronavirus (2019-nCoV) was negative;

5.3 Negative: If the test results of the FAM channel and VIC channel of the sample to be tested are Ct ≥ 45 or no Ct detection, the results were judged to be negative for 2019 novel coronavirus (2019-nCoV).

6. Quality Control

6.1 NC (ORF1ab/N) negative quality control: FAM and VIC detection channels had no obvious amplification curves, but Cy5 channel had obvious amplification curves;

6.2 NC (ORF1ab/N) positive quality control: FAM and VIC detection channels had obvious amplification curves and Ct value <_ 32, and Cy5 channel had an amplification curve or no;

The experiment was valid if all the above conditions are met at the same time. Otherwise, all the experiments should be repeated.

7. Interpretation of test results

7.1 Negative quality control products and positive quality control products need to be tested for each experiment, and the test results could only be judged when the results of the quality control products meet the quality control requirements.

7.2 When the detection channel of FAM and VIC was positive, the result of Cy5 channel (internal standard channel) may be negative due to the competition of the system.

7.3 When the internal standard result was negative, if the FAM and VIC detection channels of the detection tube were also negative, it means that the system is inhibited or the operation is wrong. The test is invalid, and the sample needs to be re-examined.

7.4 The following formats of the report were recommended:

The negative result were reported in the following format: 2019 Novel Coronavirus (2019-nCoV) RNA was not detected in the sample, and the concentration was lower than the sensitivity of the kit;

The positive result were reported in the following format: 2019 novel coronavirus (2019-nCoV) RNA was detected in the sample.

7.5 Experimental results showed that the kit of the present invention had a good detection effect, and the detection results were basically not much different from ordinary fluorescent PCR. The detection limit concentration was 300 copies/ml.

The beneficial effects of the present invention include:

(1) Using the kit and detection method of the present invention, based on thermal convection PCR, the ultra-fast PCR amplification detection of 50 cycles can be completed in 18 minutes, and the detection sensitivity is equivalent to that of ordinary fluorescent quantitative PCR, and the sensitivity can reach 300copies/ml. Combined with the direct expansion cracking method, the overall detection time does not exceed 30 minutes, which is about 1.5 hours faster than the existing known rapid detection equipment.

(2) The present invention can detect novel coronavirus-infected patients with high efficiency, high specificity and low cost by targeted detection of the N gene and ORF1ab gene of the novel coronavirus. It also prevents false negatives that may occur due to virus variability, and confirms missed detections that may be caused by mutations, which significantly improves the accuracy of virus identification.

(3) The detection kit of the present invention also includes a novel coronavirus N gene and ORF1ab gene nucleic acid target detection system and an endogenous internal standard detection system. And the present invention is a multiplex fluorescence detection kit, which can monitor the specimen collection, nucleic acid extraction process and PCR amplification process to reduce the occurrence of false negative results.

(4) After multiple rounds of screening and verification, the present invention obtains a primer-probe combination with high sensitivity, strong specificity and good repeatability from a large number of primer-probe combinations, which is suitable for thermal convection PCR and ordinary fluorescent quantitative PCR, and capable of multiple detection.

The present invention is suitable for the detection of novel coronavirus 2019-nCoV nucleic acid, and can provide a reliable basis for virus identification and prevention and control, and is worthy of popularization and application. In addition, the method of the present invention is also suitable for non-diagnostic purposes. For example, in the epidemic prevention and control process, the detection method of the present invention can be used to detect viral nucleic acids in the environment, and these viral nucleic acid information can be used for public health management.

The present invention will be further described in detail below in conjunction with specific embodiments. It should be understood that these examples are only used to illustrate the present invention and not to limit the scope of the present invention. The experimental methods without detailed conditions in the following examples are generally in accordance with the conditions described in the conventional conditions such as Sambrook. J et al. "Guide to Molecular Cloning Laboratory" (translation by Huang Peitang et al., Beijing: Science Press, 2002), or as recommended by the manufacturer. Unless otherwise stated, percentages and parts are calculated by weight. Unless otherwise specified, the experimental materials and reagents used in the following examples can be obtained from commercially available channels.

**Example 1. Design of Primers and Probes**

Based on the sequence of the novel coronavirus, the conserved regions of its genome were analyzed. Dozens of specific primers and probe sequences targeting the N gene and ORF1ab gene of the novel coronavirus were designed in these conserved regions. In addition, in order to monitor the process of sample collection, nucleic acid extraction and PCR amplification, internal standard primers and probes were designed.

In the design process of the above-mentioned primers and probes, the formation of hairpin structures, intra-primer dimers, inter-primer dimers and mismatches needs to be avoided as much as possible.

In addition, the above-designed novel coronavirus-specific primers and probe sequences were analyzed by aligning the NCBI Blast online database(https://blast.ncbi.nlm.nih.gov/Blast.cgi)to avoid non-specific binding with other viruses or human genes.

After multiple rounds of screening and optimization by conventional real-time fluorescent quantitative PCR, and then verified by thermal convection PCR, a set of primers and probe sequences with optimal sensitivity and specificity were finally determined.

Table 1 Sequences of primers and probes for novel coronavirus

| Site | Name | Sequences | SEQ ID Nos. |
|---|---|---|---|
| N gene | nCoV-N-F | GGGGAACTTCTCCTGCTAGAAT | 1 |
| | nCoV-N-R | CAGACATTTTGCTCTCAAGCTG | 2 |
| | nCoV-N-P | TTGCTGCTGCTTGACAGATT | 3 |
| | | | |
| ORF1 ab gene | nCoV-ORF1ab-F | CCCTGTGGGTTTTACACTTAA | 4 |
| | nCoV-ORF1ab-R | ACGATTGTGCATCAGCTGA | 5 |
| | nCoV-ORF1ab-P | CCGTCTGCGGTATGTGGAAAGGTTATGG | 6 |
| | | | |
| Internal standard | F7 | ACACCTCTGGATGCCACT | 7 |
| | R7 | CTCCGTACACTCTCCTACCCTC | 8 |
| | P7 | CCATTGCCAGTCCGCCGTC | 9 |

Among them, the 5'-terminal fluorescent group of nCoV-N-P is FAM, and the 3'-terminal quenching group is BHQ1; the 5'-terminal fluorescent group of nCoV-ORF1ab-P is VIC, and the 3'-terminal quenching group is BHQ1;the 5'-terminal fluorescent group of P7 is CY5, and the 3'-terminal quenching group is BHQ2.

Table 2 Components of the kit

| Component name | | Specification | Quantity | Main ingredient |
|---|---|---|---|---|
| PCR detection reagent | NC(ORF1ab/N) PCR reaction solution A | 290μl/tube | 1 | Specific primers and probes (SEQ ID NOs. 1-9), Tris-HCl buffer, MgCl₂, dNTPs |
| | NC(ORF1ab/N) PCR reaction solution B | 80μl/tube | 1 | Hot-start Taq enzyme, C-MMLV enzyme |
| Quality Control | NC(ORF1ab/N) Negative Quality Control | 200μl/tube | 1 | Pseudovirus with internal standard fragment |
| | NC(ORF1ab/N) Positive Quality Control | 200μl/tube | 1 | Pseudoviruses containing target fragments, Pseudoviruses containing internal standard fragments |

wherein the sequence of the N gene target fragment is as follows:

ATCACGTAGTCGCAACAGTTCAAGAAATTCAACTCCAGGCAGCAGTA GGGGAACTTCTCCTGCTAGAATGGCTGGCAATGGCGGTGATGCTGCTCTT GCTTTGCTGCTGCTTGACAGATTGAACCAGCTTGAGAGCAAAATGTCTGG TAAAGGCCAACAACAACAAGGCCAAACTGTCACTAAGAAATCTGCTGCT GAGGCTTCTAAGAAGCCTCGGCAAAAACGTACTGCCACTAAAGCATACA ATGTAACACAAGCTTTCGGCAGACGTGGTCCAGAACAAACCCAAGGAAA TTTTGGGGACCAGGAACTAATCAGACAAGGAACTGATTACAAACATTGGC CGCAAA(SEQ ID NO.19),

the sequence of the ORF 1ab gene target fragment is as follows:

GGATTTTGTGACTTAAAAGGTAAGTATGTACAAATACCTACAACTTG TGCTAATGACCCTGTGGGTTTTACACTTAAAAACACAGTCTGTACCGTCT GCGGTATGTGGAAAGGTTATGGCTGTAGTTGTGATCAACTCCGCGAACCC ATGCTTCAGTCAGCTGATGCACAATCGTTTTTAAACCGGGTTTGCGGTGT AAGTGCAGCCCGTCTTACACCGTGCGGCACAGGCACTAGTACTGATGTCG TATACAGGGCTTTTGACATCTACAATGATAAAGTAGCTGGTTTTGCTAAA TTCCTAAAAACTAATTGTTGTCGCTTCCAAGAAAAGGACGAAGATGACAA TTT(SEQ ID NO.20),

and the sequence of the internal standard fragments is as follows:

GGTGCCCACCAGCAGGATGGGCACATCAGGGCAGTGGTGGCACACC TCTGGATGCCACTTGTGCCGCACGTTCTCATAGGACGGCGGACTGGCAAT GGAGAAACAGATGACGAAAACGTTGGTCTGAGGGTAGGAGAGTGTACGG AGGCGGTCATACTCCTCCTGGCCCGCAGTGTCCCACAGGTTCAGGTTCAC TGTGCGCCCGTCAACTGCGCTCTGCGCGCTGTAATTGTCGAACACGGTGG GGATGTACTCTTTGGGGAAAGCGTTAGTTGTGTAGCAGATGAGCAGGCAC GTCTTGCCCACAGCCCCATCACCCACCACCACGCACTTGATGCTCTGCAT CGTGGGTGCAGTTGCTGTAGTGGAGGCAGTGCCTCC(SEQ ID NO.21).

## Example 2. Detection Method and System

In order to determine the detection system of primers and probes, the influences of different concentrations of primers and probes on the fluorescent PCR reaction were respectively tried.

The results showed that when the nCoV-N-F, nCoV-N-R primer concentrations were 20pmol, the nCoV-N-P probe concentration was 6pmol, the nCoV-ORF1ab-F, nCoV-ORF1ab-R primer concentrations were 15pmol, the nCoV-ORF1ab-P probe concentration was 6pmol, the F7, R7 primer concentrations were 5pmol, the P7 probe concentration was 3pmol, the amplification curve was the best.

The finalized reaction system is as follows:

| | |
|---|---|
| PCR reaction solution A | 12μL |
| PCR reaction solution B | 3μL |
| Nucleic acid of the sample to be tested | 5μL |

Among them, PCR reaction solution A contains specific primers and probes (SEQ ID NOs.:1-9), tris-hydrochloric acid buffer, $MgCl_2$, dNTPs (4mmol);

PCR reaction solution B contains Hot-start Taq enzyme, Reverse transcriptase (C-MMLV enzyme). Hot-start Taq enzyme, Reverse transcriptase, and dNTPs can all use commercially available products, such as Qiagen's products. In each PCR reaction solution B, the amount of Hot-start Taq enzyme is 24U, the amount of Reverse transcriptase is 24U.

## Example 3. Sensitivity Test

The virus-like particles containing target fragments were constructed. After the determination of the concentration, the pseudovirus was diluted to an appropriate concentration, and then 10-fold specific dilution was performed, and the concentrations were 3.00E+06, 3.00E+05, 3.00E+04, 3.00E+03, 3.00E+02copies/ml.

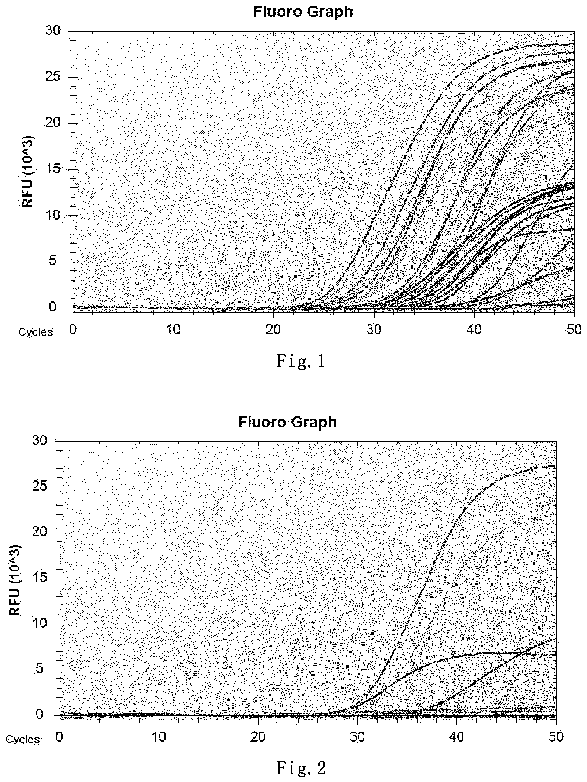

The above pseudoviruses were detected using the detection system and cycling parameters determined above. Part of the detection results refer to Fig. 1. The results showed that the detection method of the present invention had a high sensitivity, which can reach 300 copies/ml.

## Example 4. Specificity Test

Influenza A/B virus, Coronavirus 229E, Coronavirus NL63, Coronavirus OC43, Coronavirus HKU1, Respiratory syncytial virus, Adenovirus, Mycoplasma pneumoniae, and Chlamydia pneumoniae were used as specific samples for detection.

The test results were shown in Fig. 2, which showed that the detection results of the specific reference products (Influenza A/B virus, Coronavirus 229E, Coronavirus NL63, Coronavirus OC43, Coronavirus HKU1, Respiratory syncytial virus, Adenovirus, Mycoplasma pneumoniae, Chlamydia pneumoniae) were all negative, and the negative quality control was normally detected, indicating that the kit of the invention had good specificity

## Example 5. Repeatability Test

Pseudoviruses with concentrations of 1.00E+05copies/ml, 1.00E+04copies/ml and 1.00E+03copies/ml were selected for detection, and repeated tests were carried out using the detection system and cycle parameters determined above.

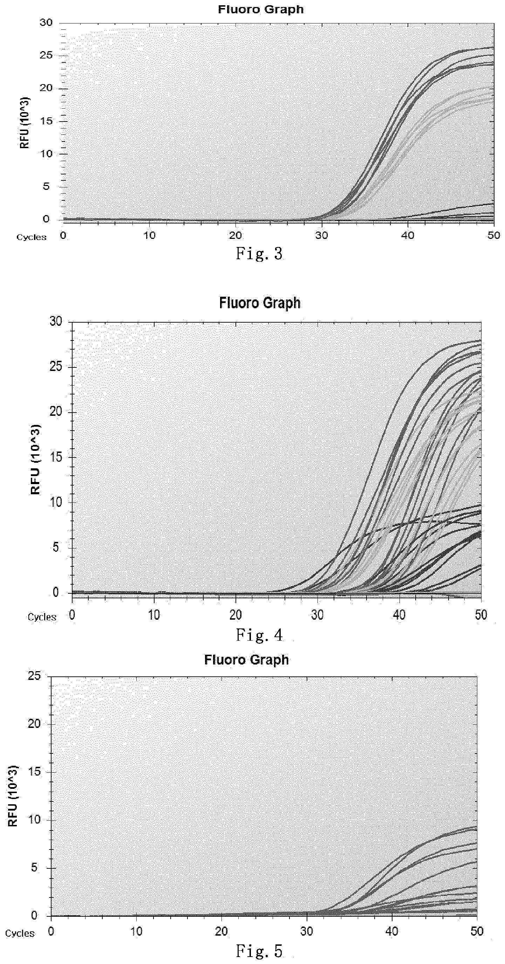

Refer to Fig. 3 (1.00E+04copies/ml) for the detection results, the results showed that the kit and detection method of the present invention had good repeatability.

## Example 6. Clinical Samples Detection

1. Sample Processing and Nucleic Acid Extraction

It was recommended to take 200μl liquid sample for nucleic acid extraction. Nucleic acid extraction or purification kits (Magnetic Bead Method) produced by Sun Yat-Sen University Daan Gene Co., Ltd. can be used (Yuesuixiebei No. 20170583 and Yuesuixiebei No. 20150302), and other commercial products can also be used, and the specific operation steps should be carried out according to the instructions of the kit.

Both the negative and positive quality controls in this kit are involved in the extraction.

2. Preparation of PCR reagent

Take out NC(ORF1ab/N) PCR reaction solution A and NC(ORF1ab/N) PCR reaction solution B from the kit, thaw at room temperature, shake and mix well, and centrifuge at 8000rpm for a few seconds before use.

Take N+1 (N=number of samples to be tested + NC(ORF1ab/N) negative quality control + NC(ORF1ab/N) positive quality control) PCR reaction tubes.

The single-person amplification system is prepared in the following table:

| NC(ORF1ab/N) PCR reaction solution A | NC(ORF1ab/N) PCR reaction solution B | Amplification system |
|---|---|---|
| 12 $\mu$l | 3 $\mu$l | 15 $\mu$l |

Take N+1 dedicated PCR reaction tubes, and use a narrow-mouth pipette tip to aliquot the PCR reagents (15 $\mu$l per tube). After the aliquoting is completed, use a centrifuge at 8000 rpm for 1 min to allow the PCR reaction solution to flow into the bottom of the PCR reaction tube.

3.Add Sample

The PCR reaction tubes were added with the extracted DNA samples, negative quality control products and positive quality control products of 5$\mu$l respectively. Cover the tube cap tightly and centrifuge at 8000 rpm for 3 minutes. After centrifugation, observe whether there are visible bubbles remaining in the reaction tube. Repeat centrifugation if necessary. If there are no visible bubbles, each reaction tube was placed into a portable rapid PCR instrument (Achem Biosystems (Korea)).

4. PCR Amplification

The program was set as follows: first perform reverse transcription at 50°C for 4 minutes, and then perform 50 cycles of thermal convection PCR reaction at 95°C.
Select the FAM channel, VIC channel, and CY5 channel for detection.

5. Result Analysis and Judgment

When the instrument was normal, and the NC (ORF1ab/N) positive quality control and NC (ORF1ab/N) negative quality control were both normal, the result analysis was performed.

FAM channel and VIC channel:

5.1 Positive: If test results of the FAM channel and VIC channel of the sample to be tested were Ct <_ 40, and the curve was S-shaped or there was an obvious exponential growth period, or the FAM channel and VIC have one Ct ≤ 40 and the other Ct ≤ 45, it was judged as 2019 novel coronavirus ( 2019-nCoV) positive;
5.2 Suspicious: The test results of the FAM channel and VIC channel of the sample to be tested are both 40≤Ct≤45. At this time, the sample should be tested repeatedly. If the Ct value of the rereading result still exists in the same range, the curve was S-shaped or has an obvious exponential expansion period, it is judged that the 2019 novel coronavirus (2019-nCoV) was positive, otherwise the 2019 novel coronavirus (2019-nCoV) was negative;
5.3 Negative: If the test results of the FAM channel and VIC channel of the sample to be tested are Ct ≥ 45 or no Ct detection, the results were judged to be negative for 2019 novel coronavirus (2019-nCoV).

6. Quality Control

6.1 NC (ORF1ab/N) negative quality control: FAM and VIC detection channels had no obvious amplification curves, but Cy5 channel had obvious amplification curves;

6.2 NC (ORF1ab/N) positive quality control: FAM and VIC detection channels had obvious amplification curves and Ct value $\leq$ 32, and Cy5 channel had an amplification curve or no;

The experiment was valid if all the above conditions are met at the same time. Otherwise, all the experiments should be repeated.

7. Interpretation of test results

7.1 Negative quality control products and positive quality control products need to be tested for each experiment, and the test results could only be judged when the results of the quality control products meet the quality control requirements.

7.2 When the detection channel of FAM and VIC was positive, the result of Cy5 channel (internal standard channel) may be negative due to the competition of the system.

7.3 When the internal standard result was negative, if the FAM and VIC detection channels of the detection tube were also negative, it means that the system is inhibited or the operation is wrong. The test is invalid, and the sample needs to be re-examined.

7.4 The following formats of the report were recommended:

The negative result report were reported in the following format: 2019 Novel Coronavirus (2019-nCoV) RNA was not detected in the sample, and the concentration was lower than the sensitivity of the kit;

The positive result report were reported in the following format: 2019 novel coronavirus (2019-nCoV) RNA was detected in the sample.

7.5 Experimental results showed that the kit of the present invention had a good detection effect, and the detection results were basically not much different from ordinary fluorescent PCR. The detection limit concentration was 1000 copies/ml.

Among the 32 suspected clinical samples tested, a total of 24 novel coronavirus 2019-nCoV nucleic acid positive clinical samples were detected. Typical test results are shown in Fig. 5.

Sequencing verification results showed that the detection accuracy of the detection system of the present invention reached 100%, which further proved the accuracy of clinical detection of the system of the present invention.

The kit of the invention has good detection effect, and the detection result is not much different from that of ordinary fluorescent PCR.

**Comparative Example 1**

In the research process, the present inventor screened dozens of PCR primers and probes for the target nucleic acid sequence of the novel coronavirus. After extensive testing, finally obtained a combination of primers and probes that can meet the needs of clinical detection with sensitivity and specificity, is suitable for thermal convection PCR, and can perform multiple detection.

For the detection target of the N gene detection target of novel coronavirus, the inventors have undergone a lot of screening and combination. Some typical primer sequences designed are as follows:

Control upstream primer nCoV-N-F2: CTAAGAAGCCTCGGCAAAA (SEQ ID NO. 10)
Control downstream primer nCoV-N-R2: CGTCTGCCGAAAGCTTG (SEQ ID NO.11)
Control probe nCoV-N-P2: TTACATTGTATGCTTTAGTGGCAGT (SEQ ID NO.12)
Control upstream primer nCoV-N-F3: GACCCCAAAATCAGCGAAAT (SEQ ID NO.13)
Control downstream primer nCoV-N-R3: TCTGGTTACTGCCAGTTGAATCTG (SEQ ID NO.14)
Control probe nCoV-N-P3: ACCCCGCATTACGTTTGGTGGACC (SEQ ID NO.15)
Control upstream primer nCoV-N-F4: TTACAAACATTGGCCGCAAA (SEQ ID NO.16)
Control downstream primer nCoV-N-R4: GCGCGACATTCCGAAGAA (SEQ ID NO.17)
Control probe nCoV-N-P4: ACAATTTGCCCCCAGCGCTTCAG (SEQ ID NO. 18)

Firstly, the conventional fluorescent quantitative PCR detection was used for primary screening.

The detection results of nCoV-N-F2 and nCoV-N-R2 were shown in Fig. 5 and the detection results indicate that the primer pair had poor specificity.

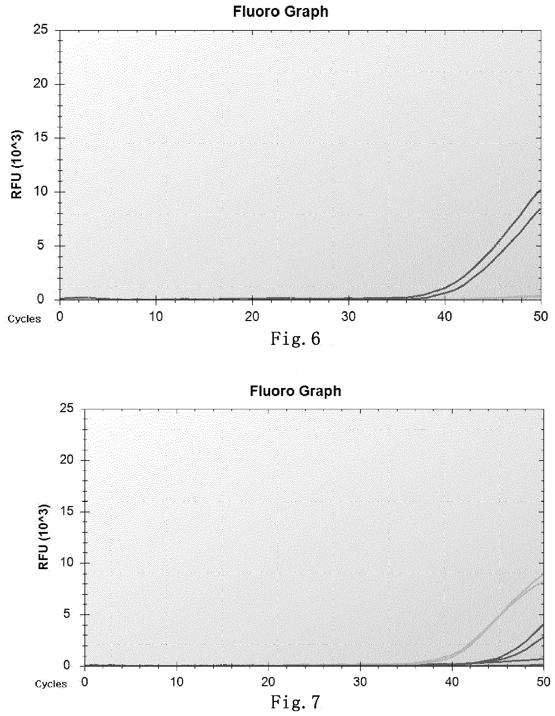

The detection results of nCoV-N-F3 and nCoV-N-R3 showed that the primer pair had better specificity and sensitivity to the N gene target nucleic acid in a single detection system. However, in the multiplex detection system, the amplification of low concentration nucleic acid of N gene was significantly inhibited. The results of single and multiplex systems tests were shown in Fig. 6 and Fig. 7. This indicated that the control primer pairs nCoV-N-F3 and nCoV-N-R3 cannot be used in multiplex detection systems.

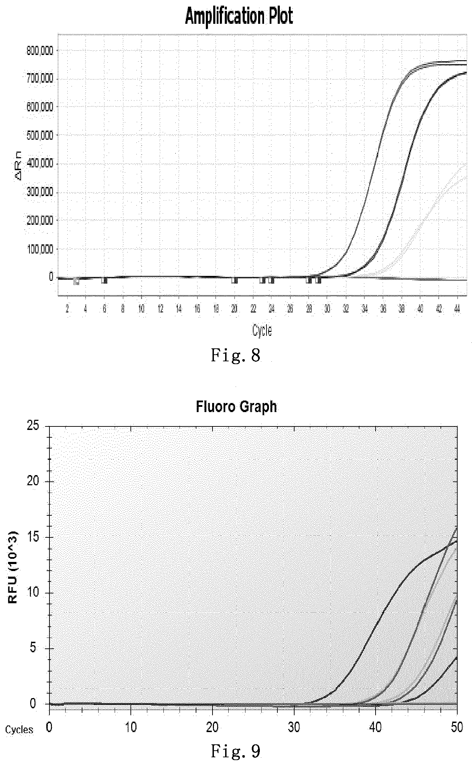

The detection results using nCoV-N-F4 and nCoV-N-R4 showed that the primer pair could perform multiple detection in the conventional fluorescent quantitative PCR detection system, and had better specificity and sensitivity. However, in thermal convection PCR, the sensitivity was significantly reduced. It showed that the control primer pair nCoV-N-F4 and nCoV-N-R4 could not be applied to the thermal convection PCR detection system. Fig. 8 and Fig. 9 show the detection results of nCoV-N-F4 and nCoV-N-R4 in the ordinary fluorescent system (Fig. 8) and the thermal convection fast system (Fig. 9) respectively.

All literatures mentioned in the present application are incorporated herein by reference, as though each one is individually incorporated by reference. In addition, it should also be understood that, after reading the above teachings of the present invention, those skilled in the art can make various changes or modifications, equivalents of which falls in the scope of claims as defined in the appended claims.

**Claims**

1. A kit for detection of novel coronavirus nucleic acid, which comprises a primer pair set;

    wherein the primer pair set comprises a first primer pair and a second primer pair;
    wherein the first primer pair comprises:

        a forward primer as shown in SEQ ID NO.1 and a reverse primer as shown in SEQ ID NO.2,
        and the second primer pair comprises:
        a forward primer as shown in SEQ ID NO.4 and a reverse primer as shown in SEQ ID NO.5.

2. The kit of claim 1, wherein the kit further comprises a probe set; wherein the probe set comprises the first probe whose nucleotide sequence is as shown in SEQ ID NO.3 and the second probe whose nucleotide sequence is shown in SEQ ID NO.6.

3. The kit of claim 1, wherein the primer pair set further comprises an internal standard primer pair, and the internal standard primer pair comprises:
   a forward primer as shown in SEQ ID NO.7 and a reverse primer as shown in SEQ ID NO.8.

4. The kit of claim 2, wherein the probe set comprises an internal standard probe whose nucleotide sequence is shown in SEQ ID NO.9.

5. The kit of claim 1, wherein the kit includes a first container, and the first container contains a primer-probe mixture, and the primer-probe mixture contains polynucleotides whose sequences are shown in SEQ ID NOs.1-6.

6. The kit of claim 5, wherein the primer-probe mixture further comprises polynucleotides whose sequences are shown in SEQ ID NOs.7-9.

7. The kit of claim 5, wherein the kit further includes a second container, and the second container contains a PCR enzyme system including a Hot-start enzyme and a Reverse transcriptase C-MMLV.

8. The kit of claim 7, wherein the first container further contains dNTPs.

9. A method for multiplex detection of novel coronavirus nucleic acid for non-diagnostic purposes, wherein the method comprising the steps of:

    (1) providing nucleic acid samples of the subject to be tested;
    (2) preparing PCR reaction system and perform PCR detection:

        wherein, the PCR reaction system includes: the nucleic acid sample provided in step (1), primer pair set and probe set;
        wherein the primer pair set comprises a first primer pair and a second primer pair;
        wherein the first primer pair comprises:

a forward primer as shown in SEQ ID NO.1 and a reverse prime as shown in SEQ ID NO.2, and the second primer pair comprises:

a forward primer as shown in SEQ ID NO.4 and a reverse primer as shown in SEQ ID NO.5; wherein the probe set comprises a first probe whose nucleotide sequence is shown in SEQ ID NO.3, and a second probe whose nucleotide sequence is shown in SEQ ID NO.6.

10. The method of claim 9, wherein the nucleic acid sample is derived from an environmental sample.

**Fluoro Graph**

Fig. 1

**Fluoro Graph**

Fig. 2

**Fluoro Graph**

Fig. 3

**Fluoro Graph**

Fig. 4

**Fluoro Graph**

Fig. 5

**Fluoro Graph**

Fig. 6

**Fluoro Graph**

Fig. 7

## Amplification Plot

Fig. 8

## Fluoro Graph

Fig. 9

## INTERNATIONAL SEARCH REPORT

| International application No. |
| --- |
| **PCT/CN2020/127752** |

| **A.** | **CLASSIFICATION OF SUBJECT MATTER** |
| --- | --- |

C12Q 1/70(2006.01)i; C12Q 1/686(2018.01)i; C12N 15/11(2006.01)i; C12R 1/93(2006.01)n

According to International Patent Classification (IPC) or to both national classification and IPC

| **B.** | **FIELDS SEARCHED** |
| --- | --- |

Minimum documentation searched (classification system followed by classification symbols)

C12Q, C12N, C12R

Documentation searched other than minimum documentation to the extent that such documents are included in the fields searched

Electronic data base consulted during the international search (name of data base and, where practicable, search terms used)

CNABS: 新型冠状病毒, 新冠, PCR, 热对流, 多重, N基因, ORF1ab基因, 引物, 探针, 试剂盒, 2019-nCov, SARS-CoV-2, novel corona virus, thermal convection, multiplex, N gene, ORF1ab gene, primer, probe, kit, for SEQ ID NO: SEQ ID NO: 1-9

| **C.** | **DOCUMENTS CONSIDERED TO BE RELEVANT** | |
| --- | --- | --- |

| Category* | Citation of document, with indication, where appropriate, of the relevant passages | Relevant to claim No. |
| --- | --- | --- |
| X | CN 111197112 A (GUANGZHOU ANBIPING MEDICINE TECHNOLOGY CO., LTD.) 26 May 2020 (2020-05-26)<br>  see description paragraph 62, paragraphs 64-73 | 1-2, 5-10 |
| Y | CN 111197112 A (GUANGZHOU ANBIPING MEDICINE TECHNOLOGY CO., LTD.) 26 May 2020 (2020-05-26)<br>  see description paragraph 62, paragraphs 64-73 | 3-4 |
| Y | CN 110273026 A (DA AN GENE CO., LTD. OF SUN YAT-SEN UNIVERSITY) 24 September 2019 (2019-09-24)<br>  see description, paragraphs 12-13 | 3-4 |
| X | CN 111088405 A (SUZHOU XINGZHI KANGZHONG BIOLOGICAL TECHNOLOGY CO., LTD.) 01 May 2020 (2020-05-01)<br>  see claim 1 | 1 |
| PX | CN 111705163 A (DA AN GENE CO., LTD. OF SUN YAT-SEN UNIVERSITY) 25 September 2020 (2020-09-25)<br>  see claims 1-10 | 1-10 |

| ☐ Further documents are listed in the continuation of Box C. | ☑ See patent family annex. |
| --- | --- |

| * | Special categories of cited documents: |
| --- | --- |
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
| --- | --- |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| **24 December 2020** | **05 February 2021** |

| Name and mailing address of the ISA/CN | Authorized officer |
| --- | --- |
| **China National Intellectual Property Administration (ISA/CN)**<br>**No. 6, Xitucheng Road, Jimenqiao, Haidian District, Beijing 100088**<br>**China** | |
| Facsimile No. **(86-10)62019451** | Telephone No. |

Form PCT/ISA/210 (second sheet) (January 2015)

## INTERNATIONAL SEARCH REPORT

| International application No. |
|---|
| **PCT/CN2020/127752** |

**Box No. I      Nucleotide and/or amino acid sequence(s) (Continuation of item 1.c of the first sheet)**

1.   With regard to any nucleotide and/or amino acid sequence disclosed in the international application, the international search was carried out on the basis of a sequence listing:

    a.   ☑ forming part of the international application as filed:

        ☑   in the form of an Annex C/ST.25 text file.

        ☐   on paper or in the form of an image file.

    b.   ☐ furnished together with the international application under PCT Rule 13*ter*.1(a) for the purposes of international search only in the form of an Annex C/ST.25 text file.

    c.   ☐ furnished subsequent to the international filing date for the purposes of international search only:

        ☐   in the form of an Annex C/ST.25 text file (Rule 13*ter*.1(a)).

        ☐   on paper or in the form of an image file (Rule 13*ter*.1(b) and Administrative Instructions, Section 713).

2.   ☐   In addition, in the case that more than one version or copy of a sequence listing has been filed or furnished, the required statements that the information in the subsequent or additional copies is identical to that forming part of the application as filed or does not go beyond the application as filed, as appropriate, were furnished.

3.   Additional comments:

Form PCT/ISA/210 (continuation of first sheet) (January 2015)

## INTERNATIONAL SEARCH REPORT
### Information on patent family members

International application No.

**PCT/CN2020/127752**

| Patent document cited in search report | | | Publication date (day/month/year) | Patent family member(s) | Publication date (day/month/year) |
|---|---|---|---|---|---|
| CN | 111197112 | A | 26 May 2020 | None | |
| CN | 110273026 | A | 24 September 2019 | None | |
| CN | 111088405 | A | 01 May 2020 | None | |
| CN | 111705163 | A | 25 September 2020 | None | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **ROUJIAN LU ; XIANG ZHAO ; JUAN LI.** Genomic characterisation and epidemiology of 2019 novel coronavirus: implications for virus origins and receptor binding. *Lancet,* 30 January 2020

- **SAMBROOK. J et al.** Guide to Molecular Cloning Laboratory. Science Press, 2002